# EUROPEAN PATENT APPLICATION

(11) **EP 4 775 187 A1**
(43) Date of publication of application: **15.07.2026**
(21) Application number: 25151076.4
(22) Date of filing: 09.01.2025
(51) Int. Cl.: A61G 5/10, A61B 5/00

(54) **PROMPTING METHOD AND PROMPTING DEVICE OF SITTING POSTURE ADJUSTMENT FOR A MOBILE ASSISTIVE DEVICE BACKGROUND**

(71) Applicant: Karma Medical Products Co., Ltd., Chiayi County 62144 (TW)
(72) Inventor: SELVARAJ, Rahul, 62144 Chiayi County (TW); WU, Jin-Hung, 62144 Chiayi County (TW)
(74) Representative: Lang, Christian

(57) **Abstract**

A prompting method of sitting posture adjustment for a mobile assistive device includes: receiving pressure values of pressure sensors of a pressure detection module; summing up the pressure values of all the pressure sensors to generate a total pressure value; calculating a ratio of each of the pressure values of the pressure sensors to the total pressure value as a pressure reference value; comparing, by the controller, the pressure reference value with a pressure threshold value; and, by the controller, controlling the prompting unit on the mobile assistive device to provide a different prompt according to a different comparison result between the pressure reference value and the pressure threshold value, so as to prompt whether the sitter's sitting posture should be adjusted.

## Description

### BACKGROUND

### Field of the Invention

The present invention relates to the sitting pressure monitoring technology, and more particularly to a prompting method and a prompting device of sitting posture adjustment for a mobile assistive device.

### Description of Related Art

The sitting posture and sitting time are closely related to human health. Abnormal sitting posture is likely to cause problems such as increased abdominal pressure, local compression on the buttocks, and etc. If one sits for a long time in a bad posture, the formation of diseases like bedsores or hemorrhoids will be accelerated or become worse. Therefore, pressure-reducing seat cushions or anti-bed sore seat cushions have begun to appear on the market to solve the aforementioned problems.

However, the existing pressure-reducing seat cushions or anti-bed sore seat cushions only relieve the pressure on the buttocks of the sitter through the design of materials or structures, but they cannot provide any prompts on whether the sitting posture is correct or not, for the sitter or their caregivers. As a result, the sitter has no way of knowing whether to adjust the sitting posture, leading to the continued uneven distribution of the pressure on the sitter's buttocks.

### SUMMARY

To overcome the problems of the existing technologies, the objective of the present invention is to provide a prompting method and a prompting device of sitting posture adjustment for a mobile assistive device, and the present invention utilizes a pressure detection module arranged to the mobile assistive device, to detect the pressure distribution, so as to utilize a prompting unit arranged to the mobile assistive device, to prompt whether the sitter's sitting posture should be adjusted or not.

To achieve the above objective, a prompting method of sitting posture adjustment for a mobile assistive device having a seat cushion equipped with a pressure detection module is provided by an embodiment of the present invention, the pressure detection module includes a plurality of pressure sensors, and the prompting method comprises the following steps: (A) receiving, by a controller, from the pressure detection module pressure values detected by the pressure sensors at different positions on the seat cushion; (B) summing up, by the controller, the pressure values of the pressure sensors to generate a total pressure value, and calculating a ratio of each of the pressure values of the respective pressure sensors to the total pressure value as a pressure reference value; (C) comparing, by the controller, the pressure reference value with a pressure threshold value; and (D) according to a different comparison result between the pressure reference value and the pressure threshold value, controlling, by the controller, a prompting unit disposed on the mobile assistive device to provide a different prompt to prompt whether a sitter's sitting posture should be adjusted.

Furthermore, another prompting method of sitting posture adjustment for a mobile assistive device having a seat cushion equipped with a pressure detection module is provided by an embodiment of the present invention, the pressure detection module includes a plurality of pressure sensors, and the prompting method includes the following steps: (A) receiving, by a controller, from the pressure detection module pressure values detected by the pressure sensors at different positions on the seat cushion; (B) selecting, by the controller, a maximum one from the pressure values of the pressure sensors as a pressure reference value; (C) comparing, by the controller, the pressure reference value with a pressure threshold value; and (D) according to a different comparison result between the pressure reference value and the pressure threshold value, controlling, by the controller, a prompting unit disposed on the mobile assistive device to provide a different prompt to prompt whether the sitter's sitting posture should be adjusted.

Optionally, the step (D) includes the following sub-steps: (D1) by the controller, controlling the prompting unit to provide a first prompt to prompt that the sitter's sitting posture should be adjusted, when the pressure reference value is greater than or equal to the pressure threshold value; and (D2) controlling, by the controller, the prompting unit to provide a second prompt to prompt that there is no need to adjust the sitter's sitting posture, when the pressure reference value is less than the pressure threshold value.

Optionally, after the step (C), the prompting method further includes the following steps: (E) displaying, by a user interface, prompt information corresponding to a comparison result between the pressure reference value and the pressure threshold value.

Optionally, the pressure threshold value is defined as a first abnormal threshold value, and the prompting method further includes the following steps: (F) controlling, by the controller, the prompting unit to provide a third prompt to prompt the degree of abnormality of the sitting posture when the pressure reference value is greater than or equal to a second abnormal threshold value that is greater than the first abnormal threshold value; (G) controlling, by the controller, the prompting unit to provide a fourth prompt to prompt a potential to tend towards an abnormal sitting posture, when the pressure reference value is less than the first abnormal threshold value Er but greater than or equal to an abnormal potential value that is less than the first abnormal threshold value; and (H) controlling, by the controller, the prompting unit to provide a fifth prompt to prompt a potential to tend towards an abnormal sitting posture when the pressure reference value is less than the abnormal potential value.

Optionally, after step (C), the prompting method further includes the following steps: (I) counting, by a timer, accumulation of time during which the pressure reference value is greater than or equal to the pressure threshold value and providing the accumulation of time to the controller; (J) determining, by the controller, whether the accumulation of time reaches a time threshold value; and (K) controlling, by the controller, the prompting unit to provide a fifth prompt when the accumulation of time reaches the time threshold value.

Optionally, the prompting unit includes light-emitting elements that respectively correspond to the pressure sensors, the controller controls light-emitting state of the light-emitting element corresponding to the pressure reference value according to a comparison result between the pressure reference value and the pressure threshold value, so as to prompt an orientation to which the sitting posture adjustment should be adjusted.

Furthermore, a prompting device of sitting posture adjustment for a mobile assistive device provided by an embodiment of the present invention is suitable for being connected to a pressure detection module disposed to a seat cushion of the mobile assistive device, the pressure detection module includes pressure sensors, and the prompting device includes: a prompting unit; and a controller communicable to the pressure detection module and the prompting unit, being configured to control the prompting unit by executing the prompting method mentioned above to prompt whether the sitter's sitting posture should be adjusted.

Optionally, the prompting device further includes a timer that is communicable to the controller and is configured to count an accumulation of time during which the pressure reference value is greater than or equal to the pressure threshold value and provide the accumulation of time to the controller, so that the controller compares the accumulation of time with a time threshold value, thereby changing the prompt of the prompting unit.

Optionally, the prompting device further includes a user interface that is communicable to the controller and is configured to display prompt information corresponding to the comparison result between the pressure reference value and the pressure threshold value.

### BRIEF DESCRIPTION OF THE DRAWINGS

After studying the detailed description in combination with the following accompanying drawings, other aspects and advantages of the present invention will be found:
FIG. 1 is a schematic diagram of a prompting device of sitting posture adjustment applied to a wheelchair according to an embodiment of the present invention;
FIG. 2 is a functional block diagram of a prompting device of sitting posture adjustment according to an embodiment of the present invention;
FIG. 3 is a flowchart of a prompting method of sitting posture adjustment according to an embodiment of the present invention;
FIG. 4 is a partial flowchart of a prompting method of sitting posture adjustment according to an embodiment of the present invention;
FIG. 5 is a partial flowchart of a prompting method of sitting posture adjustment according to an embodiment of the present invention;
FIG. 6A is a functional block diagram of a prompting device of sitting posture adjustment according to an embodiment of the present invention;
FIG. 6B is a functional block diagram of a prompting device of sitting posture adjustment according to an embodiment of the present invention;
FIG. 7 is a functional block diagram of a prompting device of sitting posture adjustment according to an embodiment of the present invention;
FIG. 8 is a partial flowchart of a prompting method of sitting posture adjustment according to an embodiment of the present invention;
FIG. 9 is a functional block diagram of a prompting device of sitting posture adjustment according to an embodiment of the present invention;
FIG. 10 is a functional block diagram of a prompting device of sitting posture adjustment according to an embodiment of the present invention;
FIG. 11 is a schematic diagram of a user interface according to an embodiment of the present invention;
FIG. 12 is a schematic diagram of a user interface according to an embodiment of the present invention; and
FIG. 13 is a flowchart of a prompting method of sitting posture adjustment according to an embodiment of the present invention.

### DETAILED DESCRIPTION

A prompting device of sitting posture adjustment provided by the present invention can be applied to mobile assistive devices to roughly determine the sitter's sitting posture based on the pressure distribution detection results, thereby providing corresponding prompts for the sitter or their caregivers for reference. Types of applicable mobile assistive devices include, for example but not limited to, wheelchairs or other devices which people can sit on instead of walking and that can be used as mobility aid. For brief illustration, wheelchairs will be taken as a specific example of mobile assistive devices hereinafter.

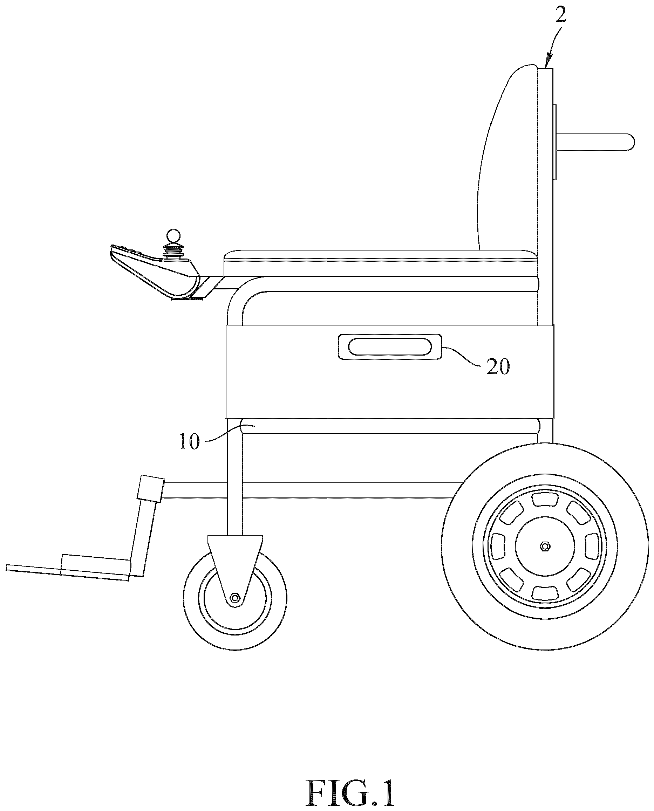

Referring to FIGs. 1 to 6, according to one embodiment of the present invention, a prompting device 1 of sitting posture adjustment for a wheelchair 2 includes, for example but not limited to, a pressure detection module 10, a prompting unit 20 and a controller 30.

The pressure detection module 10 can be disposed in or on the seat cushion of the wheelchair 2 and includes a plurality of pressure sensors 11 which is configured to detect the pressure values at different positions on the seat cushion. The pressure detection module 10 can be a flexible object that is independent of the seat cushion of the wheelchair 2 and is used to be laid on the seat cushion, such as, but not limited to, a thin sheet or a cushion body with a certain thickness; and alternatively, the pressure detection module 10 can also be integrated (embedded) into the seat cushion of the wheelchair 2. For brief illustration, in this embodiment, the pressure detection module 10 integrated into the seat cushion of the wheelchair 2 is taken as an example.

The prompting unit 20 can be disposed on the wheelchair 2, for example, but not limited to, on the side of the wheelchair 2, as shown in FIG. 1. The prompting unit 20 includes, for example, but is not limited to, at least one light-emitting element (such as, but not limited to, a light-emitting diode), a sound device (such as, but not limited to, a speaker, a buzzer or other devices that can emit sound), a display or a combination thereof.

The controller 30 can be communicable to the pressure detection module 10 and the prompting unit 20 to execute the prompting method of sitting posture adjustment for the present invention, that is, according to the results detected by the pressure detection module 10, control the prompting unit 20 to prompt the sitter whether the sitting posture should be adjusted, and prompt the effectiveness of the sitting posture adjustment.

The following demonstrates how the prompting device of the present invention executes the prompting method of sitting posture adjustment for the present invention. The prompting method includes the following steps.

Step S11: after the user activates the prompting detection function of the prompting device 1, controlling, by the controller 30, the pressure detection module 10 to make each of the pressure sensors 11 start detecting the pressure borne at the corresponding position on the seat cushion of the wheelchair 2 to generate a pressure value, and receive the pressure value provided by each pressure sensor 11 from the pressure detection module 10.

Step S12: by the controller 30, summing up all the received pressure values of the pressure sensors 11 to generate a total pressure value, and calculates the ratio of the pressure value of each pressure sensor 11 to the total pressure value as a pressure reference value.

Step S13: by the controller 30, comparing the pressure reference value corresponding to each pressure sensor 11 with a pressure threshold value.

Step S14: by the controller 30, controlling the prompting unit 20 to provide a different prompt according to a different comparison result between the pressure reference value and the pressure threshold value.

After executing Step S14, the controller 30 will return to Step S11 and repeatedly execute Steps S11 to S14 until the user turns off the prompting detection function of the prompting device 1.

In this way, the prompt of whether the sitter's sitting posture should be adjusted, or the prompt of the effectiveness of the sitter's sitting posture adjustment can be provided.

In the present invention, the setting of the magnitudes and quantity of the pressure threshold values determines the variability of the prompts that the prompting unit 20 can provide. The following are illustrative examples, but the present invention is not limited to the following examples.

### <Example 1>

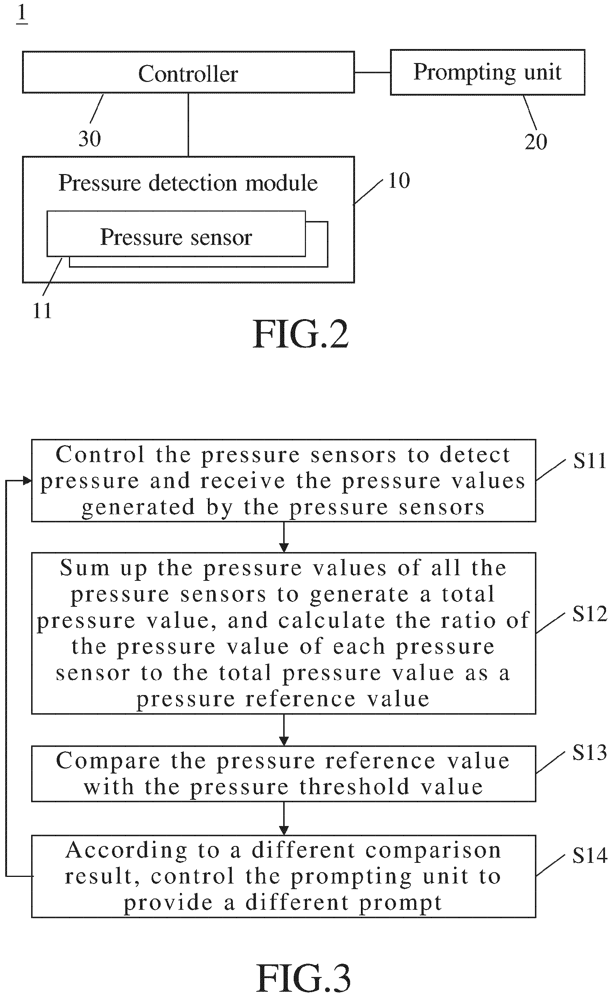

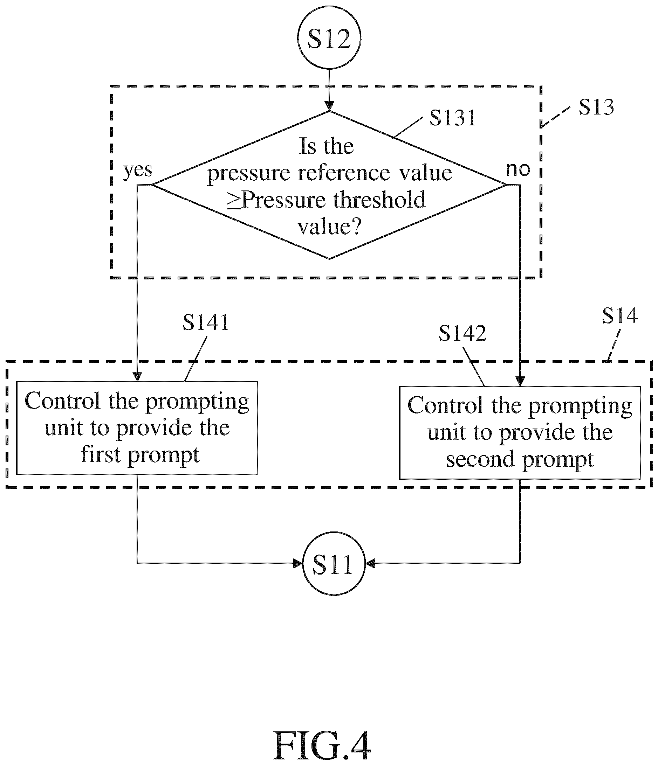

In the prompting method of sitting posture adjustment shown in FIG. 4, there is only one pressure threshold value, which represents the boundary for whether the sitting posture needs to be adjusted. Steps S13 and S14 in FIG. 3 include, for example, but not limited to, at least the following sub-steps.

Sub-step S131 of Step S13: by the controller 30, judging whether the pressure reference value is greater than or equal to the pressure threshold value. The pressure threshold value is, for example, but not limited to, 0.21.

Sub-step S141 of Step S14: controlling, by the controller 30, the prompting unit 20 to provide a first prompt to prompt that the sitter's sitting posture should be adjusted, when the pressure reference value is greater than or equal to the pressure threshold value.

Sub-step S142 of Step S14: controlling, by the controller 30, the prompting unit 20 to provide a second prompt to prompt the sitter that there is no need to adjust the sitting posture, when the pressure reference value is less than the pressure threshold value.

In this way, the user can know, from the different prompts provided by the prompting unit 20, whether the sitter's sitting posture should be adjusted. Of course, if the pressure reference value changes from being greater than or equal to the pressure threshold value to being less than the pressure threshold value, the prompt provided by the prompting unit 20 will change from the first prompt to the second prompt, so that the user can know the effectiveness of the sitting posture adjustment.

### <Example 2>

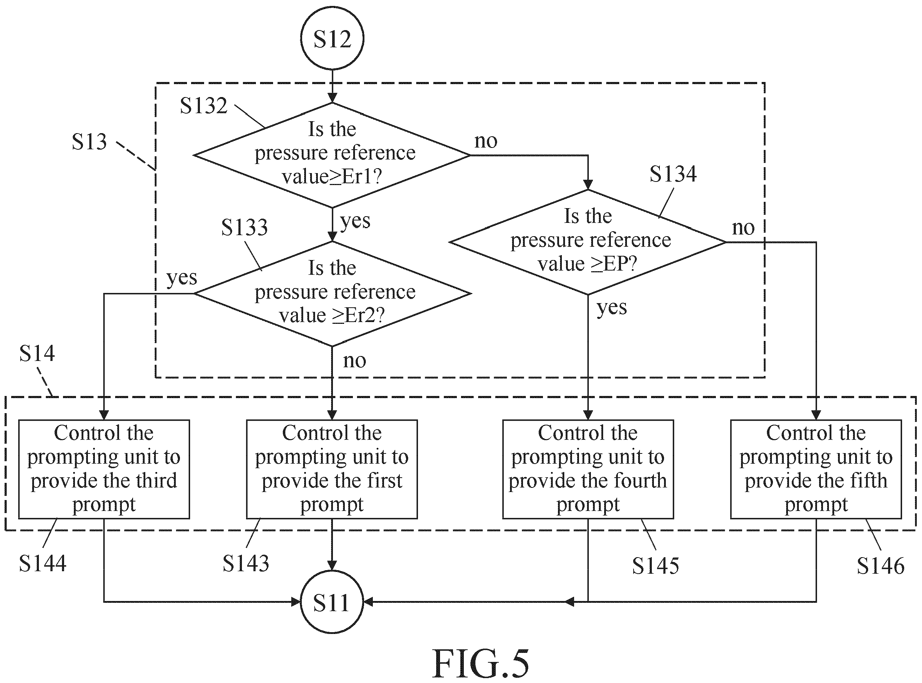

In the prompting method of sitting posture adjustment shown in FIG. 5, there are multiple pressure threshold values, such as a first abnormal threshold value Er1, a second abnormal threshold value Er2 and an abnormal potential value EP. These pressure threshold values can represent different degrees of abnormality and different degrees of approaching abnormality. Steps S13 and S14 in FIG. 3 include, for example, but not limited to, at least the following sub-steps.

Sub-step S132 of Step S13: by the controller 30, judging whether the pressure reference value is greater than or equal to the first abnormal threshold value Er1. The first abnormal threshold value Er1 is, for example, but not limited to, 0.21, which is equivalent to the pressure threshold value in sub-step S131.

Sub-step S133 of Step S13: when the pressure reference value is greater than or equal to the first abnormal threshold value Er1, it means that the sitting pressure borne at the position where the pressure sensor 11 corresponding to this pressure reference value is located, is abnormal, and such an abnormal sitting pressure represents that the sitting posture is abnormal and needs to be adjusted. At this time, the controller 30 will further judge the degree of abnormality of the sitting pressure borne at this position according to the detection result, that is, judge whether the pressure reference value is greater than or equal to the second abnormal threshold value Er2. The second abnormal threshold value Er2 is greater than the first abnormal threshold value Er1. The second abnormal threshold value Er2 is, for example, but not limited to, 0.27.

Sub-step S134 of Step S13: when the pressure reference value is less than the first abnormal threshold value Er1, it means that the sitting pressure borne at the position where the pressure sensor 11 corresponding to this pressure reference value is located, is not abnormal and is still within the acceptable range, and this situation means that there is no need to adjust the sitting posture. At this time, the controller 30 will also further judge the degree to which the sitting pressure borne at this position approaches abnormality (that is, the degree to which the sitting posture approaches abnormality) according to the detection result, that is, judge whether the pressure reference value is greater than or less than the abnormal potential value EP.

Sub-step S143 of Step S14: when the pressure reference value is greater than or equal to the first abnormal threshold value Er1 but less than the second abnormal threshold value Er2, it represents that the sitting pressure borne at the position where the pressure sensor 11 corresponding to this pressure reference value is located, is abnormal but not very serious, and this situation means that the sitting posture only needs a slight adjustment. Therefore, the controller 30 will control the prompting unit 20 to provide a second prompt for the user to know.

Sub-step S144 of Step S14: when the pressure reference value is greater than or equal to the first abnormal threshold value Er1 and greater than or equal to the second abnormal threshold value Er2, it represents that the degree of abnormality of the sitting pressure borne at the position where the pressure sensor 11 corresponding to this pressure reference value is located, is relatively higher, and this situation means that the sitting posture needs to be adjusted to a greater extent. Therefore, the controller 30 will control the prompting unit 20 to provide a third prompt for the user to know.

Sub-step S145 of Step S14: when the pressure reference value is less than the first abnormal threshold value Er1 but still greater than or equal to the abnormal potential value EP, it represents that the sitting pressure borne at the position where the pressure sensor 11 corresponding to this pressure reference value is located, is within the acceptable range but tends to be abnormal, and such situation means that attention should be paid to the sitting posture. Therefore, the controller 30 will control the prompting unit 20 to provide a fourth prompt for the user to know.

Sub-step S146 of Step S14: when the pressure reference value is less than the first abnormal threshold value Er1 and less than the abnormal potential value EP, it represents that the sitting pressure borne at the position where the pressure sensor 11 corresponding to this pressure reference value is located, is not only within the acceptable range but also has no tendency to be abnormal, and such situation means that the sitting posture only needs to be maintained. Therefore, the controller 30 will control the prompting unit 20 to provide a fifth prompt for the user to know. The first prompt and the third to fifth prompts are different from each other. The fourth prompt can be the same as or different from the first prompt mentioned above.

Optionally, the above sub-steps S133 and S144 can be omitted; and alternatively, the above sub-steps S134 and S146 can be omitted.

In this way, the user can know from the different prompt provided by the prompting unit 20 whether the sitter's sitting posture needs to adjusted, as well as know the degree of abnormality of the sitting posture and/or the potential for approaching abnormality. Of course, the change of the prompts can also let the user know the effectiveness of the sitting posture adjustment.

In the present invention, the implementation form of the prompting unit 20 also determines the variability of the prompts that the prompting unit 20 can provide. The following is an illustrative example, but the present invention is not limited to the following examples.

### <Example 3>

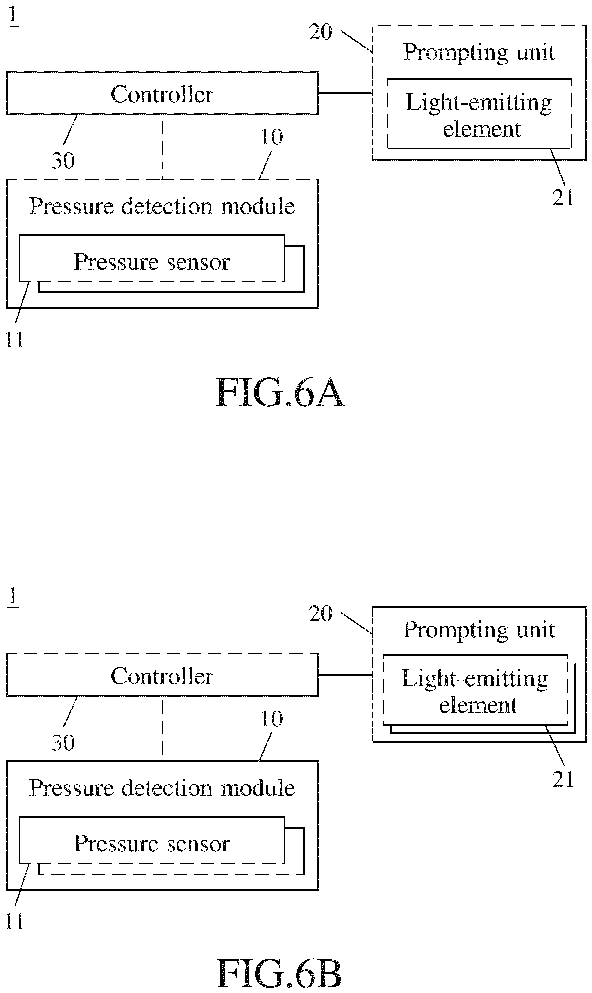

In the prompting device 1 shown in FIG. 6A, the prompting unit 20 only includes one light-emitting element 21 (such as, but not limited to, a light-emitting diode), so it can provide visual prompts to the user. This prompting unit 20 can implement the prompting method shown in FIG. 4.

Specifically, when this light-emitting element 21 changes from the off state (which can be used as the second prompt in Step S142) to the on state (which can be used as the first prompt in Step S141), it represents that the sitting posture is abnormal and needs to be adjusted. Conversely, when this light-emitting element 21 changes from the on state to the off state, it represents that the adjustment of the sitting posture is effective and the sitting posture is acceptable at this time.

### <Example 4>

In the prompting device 1 shown in FIG. 6A, the prompting unit 20 can implement the prompting method shown in FIG. 4.

Specifically, when the luminous color of the light-emitting element 21 changes from the first color (which can be used as the second prompt in Step S142) to the second color (which can be used as the first prompt in Step S141), it represents that the sitting posture is abnormal and needs to be adjusted. Conversely, when the luminous color of this light-emitting element 21 remains at the first color or changes from the second color to the first color, it represents that the sitting posture is acceptable at this time, whereby the user can know that the sitting posture does not need to be adjusted or know that the adjustment of the sitting posture is effective (that is, the effectiveness of the sitter's sitting posture adjustment is verified). The first color is different from the second color.

### <Example 5>

In the prompting device 1 shown in FIG. 6A, the prompting unit 20 can implement the prompting method shown in FIG. 5.

Specifically, when the pressure reference value is greater than or equal to the first abnormal threshold value Er1 but less than the second abnormal threshold value Er2, the luminous color of the prompting unit 20 is the third color (which can be used as the first prompt in Step S143); when the pressure reference value is greater than or equal to the second abnormal threshold value Er2, the luminous color of the prompting unit 20 is the fourth color (which can be used as the third prompt in Step S144); when the pressure reference value is less than the first abnormal threshold value Er1 but greater than or equal to the abnormal potential value EP, the luminous color of the prompting unit 20 is the fifth color (which can be used as the fourth prompt in Step S145); and when the pressure reference value is less than the abnormal potential value EP, the luminous color of the prompting unit 20 is the sixth color (which can be used as the fifth prompt in Step S146).

In this way, through the change of the luminous color of the prompting unit 20, it can be known whether the sitting posture needs to be adjusted, whether the sitting posture adjustment is effective, and the abnormal degree of sitting posture.

### <Example 6>

In the prompting device 1 shown in FIG. 6A, the prompting unit 20 can implement the prompting method of Example 6, which is similar to the prompting method shown in FIG. 5, which is done by comparing the pressure reference value with each of abnormal threshold values (such as, but not limited to, the first abnormal threshold value Er1 and the second abnormal threshold value Er2) representing different degrees of abnormality, and then determining the prompting manner of the prompting unit 20 according to the comparison result. Different from the prompting method shown in FIG. 5, the prompting method of Example 6 omits the above sub-steps S134 and S146. When the pressure reference value in sub-step S132 is less than the first abnormal threshold value Er1, sub-step S145 is directly executed.

### <Example 7>

In the prompting device 1 shown in FIG. 6A, the prompting unit 20 can implement the prompting method of Example 7, which is similar to the prompting method of Example 5 where when the pressure reference value is normal, it is further done to compare the pressure reference value with each of different abnormal potential values representing the possibility of approaching abnormality and then determine the prompting manner of the prompting unit 20 according to the comparison result. Different from the prompting method of Example 5, the prompting method of Example 7 omits the above sub-steps S133 and S144. When the pressure reference value in sub-step S132 is greater than or equal to the first abnormal threshold value Er1, sub-step S143 is directly executed.

### <Example 8>

In the prompting device 1 shown in FIG. 6B, the prompting unit 20 includes light-emitting elements 21, and these light-emitting elements 21 respectively correspond to the pressure sensors 11 of the pressure detection pad 10. Therefore, each light-emitting element 21 can implement any one of the prompting methods in Examples 3 to 7 above and provide corresponding prompts according to the change of the pressure value of the pressure sensor 11 corresponding to the light-emitting element 21. In this way, the sitter or their caregiver can know the orientation to which the sitter's sitting posture needs to be adjusted.

### <Example 9>

In the prompting device 1 shown in FIG. 6B, the prompting unit 20 includes light-emitting elements 21, and these light-emitting elements 21 respectively correspond to different abnormal threshold values, that is, the number of light-emitting elements 21 is the same as the number of abnormal threshold values. Therefore, each light-emitting element 21 can be lighted up or change its luminous color according to whether the corresponding abnormal threshold value is reached, so that the abnormal degree of sitting posture can be known.

### <Example 10>

In the prompting device 1 shown in FIG. 6B, the prompting unit 20 includes ight-emitting elements 21. One of the light-emitting elements 21 corresponds to an abnormal threshold value, and the remaining light-emitting elements 21 respectively correspond to different abnormal potential values. Therefore, each light-emitting element 21 can change its luminous color according to whether the corresponding abnormal threshold value or abnormal potential value is reached, so that it can be known whether the sitting posture is abnormal, and the degree of approaching abnornal sitting posture.

### <Example 11>

In the prompting device 1 shown in FIG. 6B, the prompting unit 20 includes light-emitting elements 21. These light-emitting elements 21 respectively correspond to different abnormal threshold values and different abnormal potential values. Therefore, each light-emitting element 21 can change its luminous color according to whether the corresponding abnormal threshold value or abnormal potential value is reached, so that the abnormal degree of sitting posture and the degree of approaching abnormal sitting posture can be known.

### <Example 12>

In the prompting device 1 of sitting posture adjustment shown in FIG. 6B, the prompting unit 20 includes two light-emitting elements 21. These two light-emitting elements 21 respectively correspond to the pressure sensors 11 located on opposite sides (left and right sides or front and back sides) of the seat cushion, and the individual control methods of these two light-emitting elements 21 can refer to the manner of any one of Examples 3 to 7 above.

In this way, the user can know whether the sitter's sitting posture needs to be adjusted, the orientation to which the sitting posture needs to be adjusted, and the effectiveness of the sitting posture adjustment.

### <Example 13>

In the prompting device 1 of sitting posture adjustment shown in FIG. 6B, the prompting unit 20 includes four light-emitting elements 21. These four light-emitting elements 21 respectively correspond to the pressure sensors 11 located around the seat cushion (left and right sides and front and back sides), and the individual control methods of these four light-emitting elements 21 can refer to the manner of any one of Examples 3 to 7 above.

In this way, the user can know whether the sitter's sitting posture needs to be adjusted, the orientation to which the sitting posture needs to be adjusted, and the effectiveness of the sitting posture adjustment.

### <Example 14>

In the example where the prompting unit 20 contains a sound device (not shown), the sound device can emit different sound effects, voices or music through the prompting manners of Example 1 or 2 above, so that the detection results of the pressure detection module 10 can have corresponding prompts.

In this way, the user can know whether the sitter's sitting posture needs to be adjusted, and the effectiveness of the sitting posture adjustment through the auditory prompts, and even optionally know the orientation to which the sitting posture needs to be adjusted.

### <Example 15>

In the example where the prompting unit 20 contains at least one light-emitting element and a sound device (not shown), the at least one light-emitting element can provide visual prompts through the appropriate prompting methods in Examples 3 to 13 above, and the sound device can emit different sound effects, voices or music through the prompting methods of Example 1 or 2 above, so that the detection results of the pressure detection module 10 can have more various prompts.

### <Example 16>

In the example where the prompting unit 20 contains a display (not shown), the display can provide different visual prompts through the prompting manner of Example 1 or 2 above, such as, but not limited to, displaying the prompt information corresponding to the comparison result of the pressure reference value and the pressure threshold value.

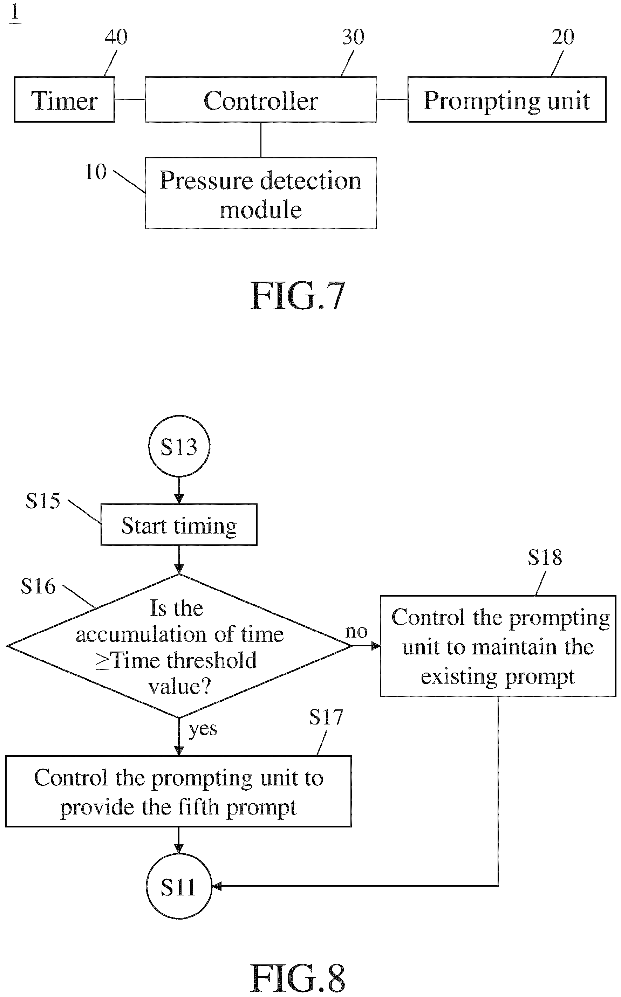

Referring to FIGs. 7 and 8, a prompting device 1 provided by an embodiment of the present invention may include a timer 40 in addition to the pressure detection module 10, the prompting unit 20 and the controller 30 shown in FIG. 2.

The timer 40 is communicable to the controller 30, and can individually count the accumulation of time in which the pressure reference value corresponding to the individual pressure sensor 11 of the pressure detection module 10 is greater than or equal to the pressure threshold value (or the first abnormal threshold value Er1) after Step S13 in FIG. 3. This accumulation of time starts from the time point at which the controller 30 judges that the pressure reference value is greater than or equal to the pressure threshold value (or the first abnormal threshold value Er1), and is the duration when the pressure reference value stays in a state of being greater than or equal to the pressure threshold value (or the first abnormal threshold value Er1).

Specifically, when the controller 30 learns that the pressure reference value corresponding to a certain pressure sensor is abnormal (that is, greater than or equal to the pressure threshold value in Step S131 or greater than or equal to the first abnormal threshold value Er1 in Step S132), the controller 30 will control the timer 40 to start timing in Step S15, and the timer 40 will also return the accumulation of time to the controller 30; and the controller 30 will judge in Step S16 whether the accumulation of time has reached (that is, is greater than or equal to) a time threshold value.

When the accumulation of time is greater than or equal to the time threshold value in Step S16, it means that the sitter's abnormal posture has maintained for a too long time without improvement. Therefore, the controller 30 will control the prompting unit 20 to provide a fifth prompt in Step S17, such as, but not limited to, changing the light-emitting state of the prompting unit 20, making the sound device emit a specific sound effect, voice or music, or making the display show specific content, and then return to Step S11 to control the pressure detection module 10 to continue detection. Changing the light-emitting state of the prompting unit 20 includes, for example, but not limited to, controlling the flashing frequency (such as starting to flash or flashing more frequently) of the prompting unit 20, or increasing the luminance of the prompting unit 20, or changing the luminous color of the prompting unit 20. The fifth prompt in Step S17 is different from the fifth prompt in the above sub-step S146, so it can be regarded as a sixth prompt.

Conversely, when the accumulation of time is less than the time threshold value in Step S16, the controller 30 will control the prompting unit 20 to continue to maintain the original prompt in Step S18, and then return to Step S11 to control the pressure detection module 10 to continue detection.

In this way, urging or intensively prompting that the sitter's sitting posture needs to be adjusted.

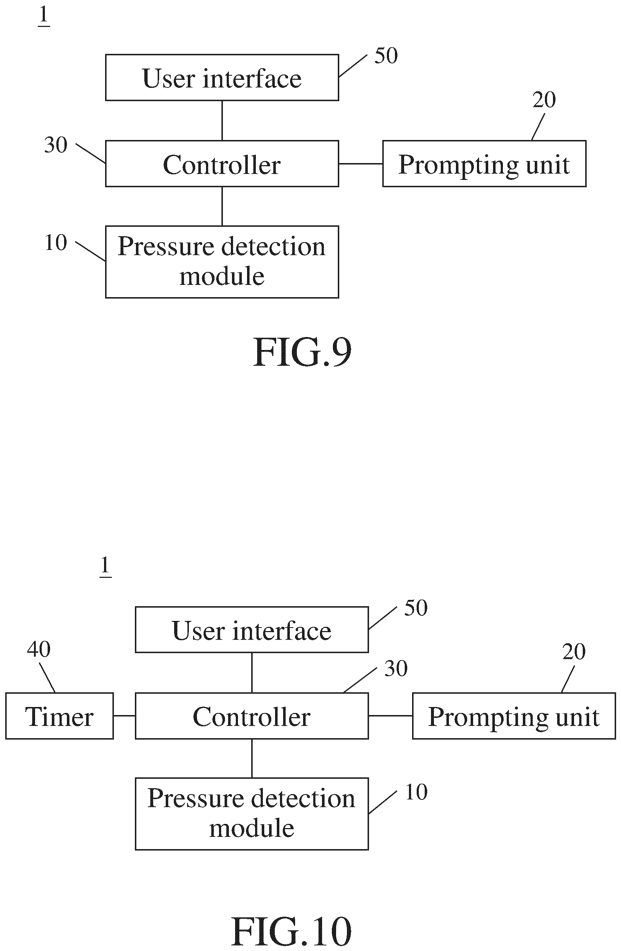

Referring to FIG. 9, a prompting device 1 provided by an embodiment of the present invention may include a user interface 50 in addition to the pressure detection module 10, the prompting unit 20 and the controller 30 shown in FIG. 2.

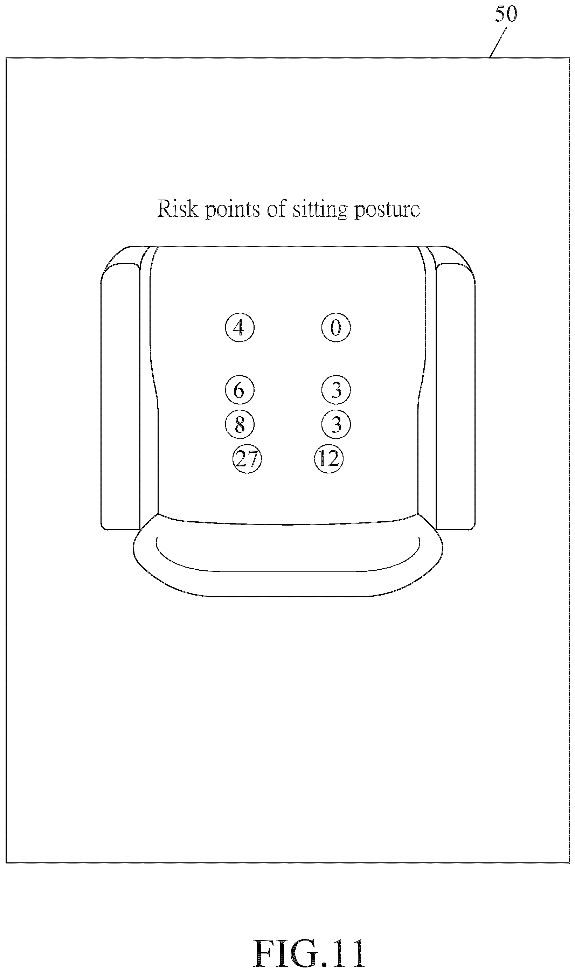

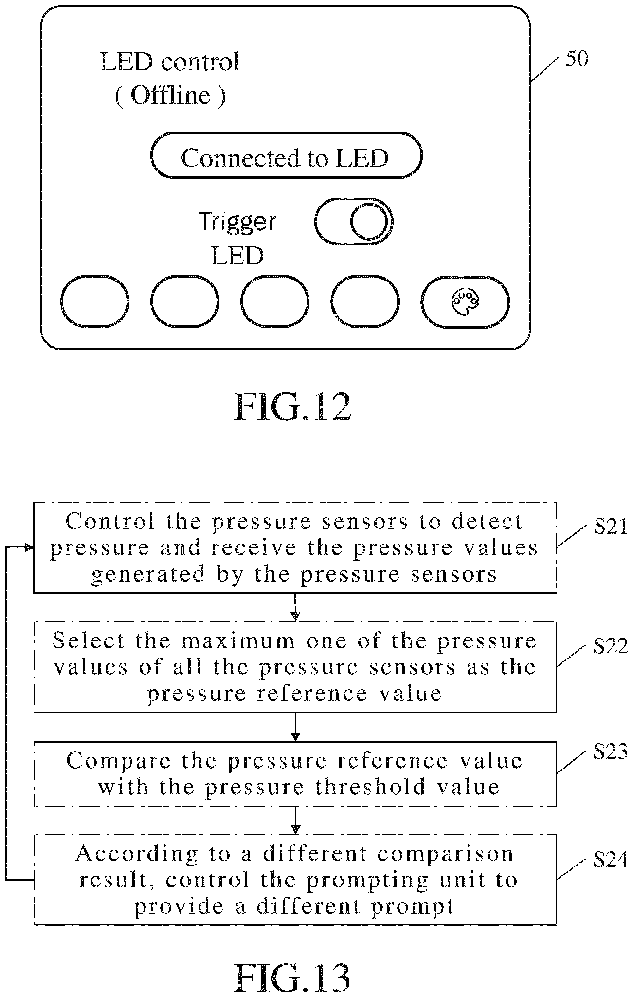

The user interface 50 is provided by, for example, but not limited to, an application program serving as a part of the prompting device 1 and installed in the computer device of the user (such as the sitter, the caregiver or other remote personnel), and is displayed on the display of the computer device. The application program can be communicable to the controller 30 through the communication module of the computer device, so that the user interface 50 can display prompt information, such as, but not limited to, the comparison result of the pressure ratio and the pressure threshold value, the pressure values of the respective pressure sensors, the pressure ratios corresponding to respective pressure sensors, the total pressure value, other information or any combination thereof. For example, the screen of the user interface 50 in FIG. 11 shows the sitting posture risk points representing the pressure ratio to remind the user. For example, the sitting posture risk point "4" represents the pressure ratio "0.4". For example, the screen of the user interface 50 in FIG. 12 shows the setting page for the prompting unit 20.

In this way, the user can know whether the sitter needs to adjust the sitting posture and verify the effectiveness of the sitting posture adjustment through the user interface 50.

Referring to FIG. 10, a prompting device 1 provided by an embodiment of the present invention further includes the timer 40 shown in FIG. 7 and the user interface 50 shown in FIG. 9, in addition to the pressure detection module 10, the prompting unit 20 and the controller 30 shown in FIG. 2. Therefore, the user interface 50 can display not only the prompt information, the pressure values of each pressure sensor, the pressure reference values corresponding to each pressure sensor, the total pressure value and any combination thereof, but also the timing results.

Referring to FIG. 13, according to an embodiment of the present invention, another prompting method of sitting posture adjustment for a mobile assistive device is also provided, is applicable to the prompting device 1 shown in FIGs. 2, 6A, 6B, 7, 9 and 10, and can implement the prompting method of any one of Examples 1 to 16 above.

In this prompting method, Step S21, Step S23 and Step S24 are respectively the same as the aforementioned Step S21, Step S13 and Step S14, so their explanations can refer to the relevant descriptions of Step S21, Step S13 and Step S14 and will not be repeated here. However, in Step S22 of this prompting method, the controller 30 selects the maximum one from the pressure values of all the pressure sensors 11 as a pressure reference value.

Therefore, the prompting method in FIG. 13 can also achieve the same or similar technical effects as the above embodiments or examples.

In the present invention, the types of wheelchairs to which the prompting device can be applied are not limited. Applicable types of wheelchairs include, for example but not limited to, electric wheelchairs, manual wheelchairs, reclining wheelchairs and non-reclining wheelchairs.

In the present invention, the prompting device can be provided with a plurality of prompting units, and these prompting units are respectively arranged on the periphery of the wheelchair to prompt which side of the wheelchair the center of gravity of the sitter sitting on the wheelchair is biased towards.

In the example where there is one prompting unit arranged on each of the left and right sides of the wheelchair and each prompting unit has only one light-emitting element, when the center of gravity of the sitter sitting on the wheelchair is biased to the left side, since the weight will be more concentrated on the left side of the pressure detection pad, the pressure values of the pressure sensors close to the left side will be relatively high, resulting in that the pressure ratios corresponding to the pressure sensors close to the left side are prone to be abnormal (that is, reaching or exceeding the pressure threshold value). Therefore, the light-emitting state of the prompting unit on the left side can be used as a prompt for the center of gravity of the sitter sitting on the wheelchair being biased to the left side. Similarly, when the center of gravity of the sitter sitting on the wheelchair is biased to the right side, the light-emitting state of the prompting unit on the right side can be used as a prompt for the center of gravity of the sitter sitting on the wheelchair being biased to the right side.

Taking the example where there is one prompting unit arranged on each of the left and right sides of the wheelchair and each prompting unit is equipped with a plurality of light-emitting elements, when the center of gravity of the sitter sitting on the wheelchair is biased to the left side, those light-emitting elements on the left side can be used as a prompt for the center of gravity of the sitter sitting on the wheelchair being biased to the left side; furthermore, through the pairing design of the light-emitting elements and the pressure sensors, those light-emitting elements on the left side can more accurately prompt the biased direction, such as, but not limited to, the front left, the back left, etc.; or, through the pairing design of the number of the lit light-emitting elements and different pressure threshold values, those light-emitting elements on the left side can prompt the degree to which the center of gravity of the sitter sitting on the wheelchair is biased to the left side. Similarly, when the center of gravity of the sitter sitting on the wheelchair is biased to the right side, the light-emitting state of the prompting unit on the right side can be used as a prompt for the center of gravity of the sitter sitting on the wheelchair being biased to the right side.

In the present invention, the prompting units of the prompting device can also be used as turn signals. For example, the light-emitting elements of the two prompting units respectively arranged on the left and right sides of the electric wheelchair can prompt the electric wheelchair to turn left or right.

In the present invention, the prompting units of the prompting device can also be used for power indication, such as, but not limited to, low voltage indication.

In the present invention, the types of pressure sensors and the units of pressure values are not limited. The unit of pressure values can be, for example, but not limited to, millimeters of mercury (mmHg).

Although the present invention has been disclosed with the above embodiments, these embodiments are not intended to limit the present invention. Any changes, modifications and combinations of various implementation forms within the spirit and scope of the present invention all fall within the patent protection scope of the present invention. For the protection scope defined by the present invention, please refer to the attached claims.

## Claims

1. A prompting method of sitting posture adjustment for a mobile assistive device (2) having a seat cushion equipped with a pressure detection module (10), the pressure detection module (10) including pressure sensors (11), and the prompting method being **characterized by** comprising the following steps:
(A) receiving, by a controller (30), from the pressure detection module (10) pressure values detected by the pressure sensors (11) at different positions on the seat cushion;
(B) summing up, by the controller (30), the pressure values of the pressure sensors (11) to generate a total pressure value, and calculating a ratio of each of the pressure values of the pressure sensors (11) to the total pressure value as a pressure reference value;
(C) comparing, by the controller (30), the pressure reference value with a pressure threshold value; and
(D) according to a different comparison result between the pressure reference value and the pressure threshold value, controlling, by the controller (30), a prompting unit (20) disposed on the mobile assistive device (2) to provide a different prompt to prompt whether a sitter's sitting posture should be adjusted.

2. A prompting method of sitting posture adjustment for mobile assistive device (2) having a seat cushion of the mobile assistive device (2) equipped with a pressure detection module (10), the pressure detection module (10) including a plurality of pressure sensors (11), and the prompting method being **characterized by** comprising the following steps:
(A) receiving, by a controller (30), from the pressure detection module (10) pressure values detected by the pressure sensors (11) at different positions on the seat cushion;
(B) selecting, by the controller (30), a maximum one from the pressure values of the pressure sensors (11) as a pressure reference value;
(C) comparing, by the controller (30), the pressure reference value with a pressure threshold value; and
(D) according to a different comparison result between the pressure reference value and the pressure threshold value, controlling, by the controller (30), a prompting unit (20) disposed on the mobile assistive device (2) to provide a different prompt to prompt whether a sitter's sitting posture should be adjusted.

3. The prompting method of sitting posture adjustment for the mobile assistive device (2) as claimed in claim 1 or 2 is **characterized in that** the step (D) includes the following sub-steps:
(D1) by the controller (30), controlling the prompting unit (20) to provide a first prompt to prompt that the sitter's sitting posture should be adjusted, when the pressure reference value is greater than or equal to the pressure threshold value; and
(D2) controlling the prompting unit (20) to provide a second prompt to prompt that there is no need to adjust the sitter's sitting posture, when the pressure reference value is less than the pressure threshold value, by the controller (30).

4. The prompting method of sitting posture adjustment for the mobile assistive device (2) as claimed in claim 1 o 2 is **characterized in that** after the step (C), the prompting method further comprises the following steps:
(E) displaying, by a user interface (50), prompt information corresponding to a comparison result between the pressure reference value and the pressure threshold value.

5. The prompting method of sitting posture adjustment for the mobile assistive device (2) as claimed in claim 3 is **characterized in that** the pressure threshold value is defined as a first abnormal threshold value (Er1), and the prompting method further comprises the following steps:
(F) by the controller (30), controlling the prompting unit (20) to provide a third prompt to prompt a degree of abnormality of sitting posture, when the pressure reference value is greater than or equal to a second abnormal threshold value (Er2) greater than the first abnormal threshold value (Er1);
(G) controlling, by the controller (30), the prompting unit (20) to provide a fourth prompt to prompt a potential to tend towards an abnormal sitting posture, when the pressure reference value is less than the first abnormal threshold value (Er1) but greater than or equal to an abnormal potential value (EP) that is less than the first abnormal threshold value (Er1); and
(H) controlling, by the controller (30), the prompting unit (20) to provide a fifth prompt to prompt a potential to tend towards an abnormal sitting posture, when the pressure reference value is less than the abnormal potential value (EP).

6. The prompting method of sitting posture adjustment for the mobile assistive device (2) as claimed in claim 1 or 2 is **characterized in that** after step (C), the prompting method further comprises the following steps:
(I) counting, by a timer (40), an accumulation of time during which the pressure reference value is greater than or equal to the pressure threshold value, and providing the accumulation of time to the controller (30);
(J) determining, by the controller (30), whether the accumulation of time reaches a time threshold value; and
(K) controlling, by the controller (30), the prompting unit (20) to provide a fifth prompt when the accumulation of time reaches the time threshold value.

7. The prompting method of sitting posture adjustment for the mobile assistive device (2) as claimed in claim 1 or 2 is **characterized in that** the prompting unit (20) includes light-emitting elements (21) that respectively correspond to the pressure sensors (11), and in the step (D), the controller (30) controls light-emitting state of the light-emitting element (21) corresponding to the pressure reference value according to a comparison result between the pressure reference value and the pressure threshold value, so as to prompt an orientation to which the sitting posture adjustment should be adjusted.

8. A prompting device of sitting posture adjustment for a mobile assistive device (2), being suitable to be connected to a pressure detection module (10) disposed to a seat cushion of the mobile assistive device (2), the pressure detection module (10) including pressure sensors (11), and the prompting device being **characterized by** comprising:
a prompting unit (20); and
a controller (30) communicable to the pressure detection module (10) and the prompting unit (20), and configured to control the prompting unit (20) by executing the prompting method in claim 1 or 2, to prompt whether the sitter's sitting posture should be adjusted.

9. The prompting device of sitting posture adjustment for the mobile assistive device (2) as claimed in claim 8 is **characterized by** further comprising a timer (40) that is communicable to the controller (30) and is configured to count an accumulation of time during which the pressure reference value is greater than or equal to the pressure threshold value, and provide the accumulation of time to the controller (30), so that the controller (30) compares the accumulation of time with a time threshold value, thereby changing the prompt of the prompting unit (20).

10. The prompting device of sitting posture adjustment for the mobile assistive device (2) as claimed in claim 8 is **characterized by** further comprising a user interface (50) that is communicable to the controller (30) and is configured to display prompt information corresponding to a comparison result between the pressure reference value and the pressure threshold value.

## Amended claims

### Amended claims in accordance with Rule 137(2) EPC.

1. A prompting method of sitting posture adjustment for a mobile assistive device (2) having a seat cushion equipped with a pressure detection module (10), the pressure detection module (10) including pressure sensors (11), and the prompting method being **characterized by** comprising the following steps:
(A) receiving, by a controller (30), from the pressure detection module (10) pressure values detected by the pressure sensors (11) at different positions on the seat cushion;
(B) summing up, by the controller (30), the pressure values of all of the pressure sensors (11) to generate a total pressure value, and calculating a ratio of each of the pressure values of the pressure sensors (11) to the total pressure value as a pressure reference value;
(C) comparing, by the controller (30), the pressure reference value with a pressure threshold value; and
(D) according to a different comparison result between the pressure reference value and the pressure threshold value, controlling, by the controller (30), a prompting unit (20) disposed on the mobile assistive device (2) to provide a different prompt to prompt whether a sitter's sitting posture should be adjusted, and prompt the effectiveness of the sitting posture adjustment;
wherein the step (D) includes the following sub-steps:
(D1) by the controller (30), controlling the prompting unit (20) to provide a first prompt to prompt that the sitter's sitting posture should be adjusted, when the pressure reference value is greater than or equal to the pressure threshold value; and
(D2) controlling the prompting unit (20) to provide a second prompt to prompt that there is no need to adjust the sitter's sitting posture, when the pressure reference value is less than the pressure threshold value, by the controller (30); and
the prompting unit (20) includes light-emitting elements (21) that respectively correspond to the pressure sensors (11), and in the step (D), the controller (30) controls light-emitting state of the light-emitting element (21) corresponding to the pressure reference value according to a comparison result between the pressure reference value and the pressure threshold value, so as to prompt an orientation to which the sitting posture adjustment should be adjusted.

2. A prompting method of sitting posture adjustment for mobile assistive device (2) having a seat cushion of the mobile assistive device (2) equipped with a pressure detection module (10), the pressure detection module (10) including a plurality of pressure sensors (11), and the prompting method being **characterized by** comprising the following steps:
(A) receiving, by a controller (30), from the pressure detection module (10) pressure values detected by the pressure sensors (11) at different positions on the seat cushion;
(B) selecting, by the controller (30), a maximum one from the pressure values of the pressure sensors (11) as a pressure reference value;
(C) comparing, by the controller (30), the pressure reference value with a pressure threshold value; and
(D) according to a different comparison result between the pressure reference value and the pressure threshold value, controlling, by the controller (30), a prompting unit (20) disposed on the mobile assistive device (2) to provide a different prompt to prompt whether a sitter's sitting posture should be adjusted and prompt the effectiveness of the sitting posture adjustment;
wherein the step (D) includes the following sub-steps:
(D1) by the controller (30), controlling the prompting unit (20) to provide a first prompt to prompt that the sitter's sitting posture should be adjusted, when the pressure reference value is greater than or equal to the pressure threshold value; and
(D2) controlling the prompting unit (20) to provide a second prompt to prompt that there is no need to adjust the sitter's sitting posture, when the pressure reference value is less than the pressure threshold value, by the controller (30); and
the prompting unit (20) includes light-emitting elements (21) that respectively correspond to the pressure sensors (11), and in the step (D), the controller (30) controls light-emitting state of the light-emitting element (21) corresponding to the pressure reference value according to a comparison result between the pressure reference value and the pressure threshold value, so as to prompt an orientation to which the sitting posture adjustment should be adjusted.

3. The prompting method of sitting posture adjustment for the mobile assistive device (2) as claimed in claim 1 or 2 is **characterized in that** after the step (C), the prompting method further comprises the following steps:
(E) displaying, by a user interface (50), prompt information corresponding to a comparison result between the pressure reference value and the pressure threshold value.

4. The prompting method of sitting posture adjustment for the mobile assistive device (2) as claimed in claim 1 or 2 is **characterized in that** the pressure threshold value is defined as a first abnormal threshold value (Er1), and the prompting method further comprises the following steps:
(F) by the controller (30), controlling the prompting unit (20) to provide a third prompt to prompt a degree of abnormality of sitting posture, when the pressure reference value is greater than or equal to a second abnormal threshold value (Er2) greater than the first abnormal threshold value (Er1);
(G) controlling, by the controller (30), the prompting unit (20) to provide a fourth prompt to prompt a potential to tend towards an abnormal sitting posture, when the pressure reference value is less than the first abnormal threshold value (Er1) but greater than or equal to an abnormal potential value (EP) that is less than the first abnormal threshold value (Er1); and
(H) controlling, by the controller (30), the prompting unit (20) to provide a fifth prompt to prompt a potential to tend towards an abnormal sitting posture, when the pressure reference value is less than the abnormal potential value (EP).

5. The prompting method of sitting posture adjustment for the mobile assistive device (2) as claimed in claim 1 or 2 is **characterized in that** after step (C), the prompting method further comprises the following steps:
(I) counting, by a timer (40), an accumulation of time during which the pressure reference value is greater than or equal to the pressure threshold value, and providing the accumulation of time to the controller (30);
(J) determining, by the controller (30), whether the accumulation of time reaches a time threshold value; and
(K) controlling, by the controller (30), the prompting unit (20) to provide a fifth prompt when the accumulation of time reaches the time threshold value.

6. A prompting device of sitting posture adjustment for a mobile assistive device (2), being suitable to be connected to a pressure detection module (10) disposed to a seat cushion of the mobile assistive device (2), the pressure detection module (10) including pressure sensors (11), and the prompting device being **characterized by** comprising:
a prompting unit (20); and
a controller (30) communicable to the pressure detection module (10) and the prompting unit (20), and configured to control the prompting unit (20) by executing the prompting method in claim 1 or 2, to prompt whether the sitter's sitting posture should be adjusted.

7. The prompting device of sitting posture adjustment for the mobile assistive device (2) as claimed in claim 6 is **characterized by** further comprising a timer (40) that is communicable to the controller (30) and is configured to count an accumulation of time during which the pressure reference value is greater than or equal to the pressure threshold value, and provide the accumulation of time to the controller (30), so that the controller (30) compares the accumulation of time with a time threshold value, thereby changing the prompt of the prompting unit (20).

8. The prompting device of sitting posture adjustment for the mobile assistive device (2) as claimed in claim 6 is **characterized by** further comprising a user interface (50) that is communicable to the controller (30) and is configured to display prompt information corresponding to a comparison result between the pressure reference value and the pressure threshold value.
